(19) Europäisches Patentamt
European Patent Office
Office européen des brevets

(11) **EP 2 857 819 A1**

(12) **EUROPEAN PATENT APPLICATION**
published in accordance with Art. 153(4) EPC

(43) Date of publication:
**08.04.2015 Bulletin 2015/15**

(21) Application number: **13800017.9**

(22) Date of filing: **12.02.2013**

(51) Int Cl.:
***G01M 11/00*** *(2006.01)* ***G01J 1/02*** *(2006.01)*
***G01J 3/50*** *(2006.01)* ***G01J 5/60*** *(2006.01)*
***A61B 3/028*** *(2006.01)* ***H05B 37/02*** *(2006.01)*

(86) International application number:
**PCT/JP2013/000734**

(87) International publication number:
**WO 2013/183195 (12.12.2013 Gazette 2013/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.06.2012 JP 2012128324**

(71) Applicant: **Panasonic Intellectual Property Management Co., Ltd.**
**Osaka 540-6207 (JP)**

(72) Inventor: **KAWAMURA, Ryo**
**Osaka-shi, Osaka 540-6207 (JP)**

(74) Representative: **Appelt, Christian W.**
**Boehmert & Boehmert**
**Anwaltspartnerschaft mbB**
**Patentanwälte Rechtsanwälte**
**Pettenkoferstrasse 20-22**
**80336 München (DE)**

# (54) ILLUMINATION EVALUATION DEVICE AND ILLUMINATION EVALUATION METHOD

(57) An illumination evaluation apparatus evaluates an illumination environment by an arbitrary light source and includes: a visual target presenting unit which presents a visual target 13 at an arbitrary test position on an irradiation area 12 irradiated by illumination light emitted from the light source, the visual target being provided for acquiring contrast sensitivity of an observer looking at the irradiation area 12; and a recording unit which acquires a value of the contrast sensitivity when the observer looks at the visual target 13 presented by the visual target presenting unit and records the acquired value of the contrast sensitivity and positional information at the test position. The illumination evaluation apparatus evaluates visibility at the arbitrary position on the irradiation area 12 by the acquired value of the contrast sensitivity.

FIG. 9

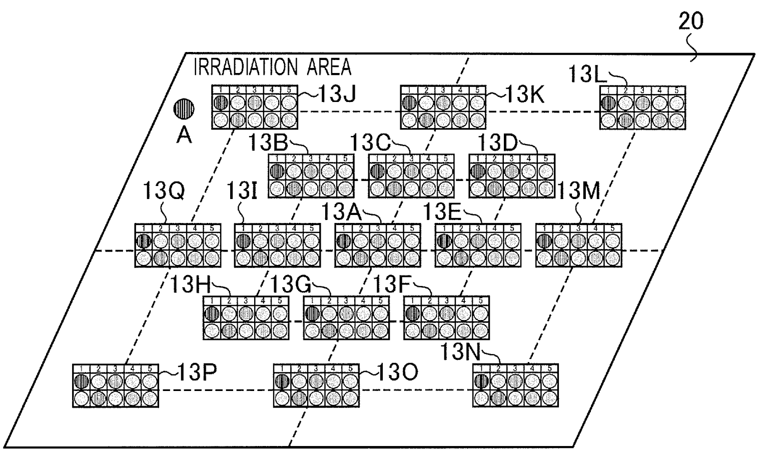


EP 2 857 819 A1

## Description

TECHNICAL FIELD

The present invention relates to an illumination evaluation apparatus and an illumination evaluation method for evaluating illumination light.

BACKGROUND ART

There are known techniques for examining an observer's vision, including techniques described in Patent Literatures 1 and 2, for example.

Patent Literature 1 describes a nighttime eye examination apparatus which presents a visual target in a dark visual field after the subject's eye is light-adapted and then measures visual recovery time taken for the subject's eye to discern the presented target. This nighttime eye examination apparatus includes: a visual target brightness changing unit configured to change the visual target brightness; and a visual target illumination unit configured to present a visual target with a low brightness as an initial value of the measurement.

Patent Literature 2 describes a nighttime vision analyzer which includes : a normal vision test means configured to measure the vision when visual targets are presented in a normal brightness area; and a nighttime vision measurement means configured to present visual targets with low brightness in a dark visual field after the subject's eye is light-adapted and then measures the time taken for the subject's eye to discern the visual target. In this nighttime vision analyzer, a target light source illuminating the visual target is composed of a white LED.

The techniques described in Patent Literatures 1 and 2 are intended to measure the time taken for a light-adapted eye to recognize a dark visual target. In the techniques described in Patent Literatures 1 and 2, moreover, the subject looks into a lens tube to recognize the visual targets. Accordingly, the techniques described in Patent Literatures 1 and 2 are not designed to evaluate brightness of illumination environments.

The illumination environment by illumination light projected from a light source is evaluated by subjective assessment of the subject, such as visibility. In this subjective assessment, the subject's psychological and physical conditions affect the evaluation results. Accordingly, the illumination environment cannot be evaluated objectively. Furthermore, this subjective assessment cannot represent illumination environments as quantified reproducible results.

Accordingly, the present invention was made in the light of the aforementioned circumstance, and an object of the present invention is to provide an illumination evaluation apparatus and an illumination evaluation method which are capable of quantitatively representing an illumination environment.

CITATION LIST

PATENT LITERATURE

Patent Literature 1: Japanese Patent No. 3623401
Patent Literature 2: Japanese Patent No. 3850186

SUMMARY OF INVENTION

An illumination evaluation apparatus according to a first aspect of the present invention is an illumination evaluation apparatus evaluating an illumination environment by at least an arbitrary light source, the apparatus including: a visual target presenting unit which presents a visual target at an arbitrary test position on an irradiation area irradiated by illumination light emitted from the at least a light source, the visual target being provided for acquiring contrast sensitivity of an observer looking at the irradiation area; and a recording unit which acquires a value of the contrast sensitivity when the observer looks at the visual target presented by the visual target presenting unit. The acquired value of the contrast sensitivity is used to evaluate visibility at the arbitrary position on the irradiation area.

An illumination evaluation apparatus according to a second aspect of the present invention is the illumination evaluation apparatus according to the first aspect, in which the visual target presenting unit presents the visual target for acquiring the contrast sensitivity at each of a plurality of arbitrary positions on the irradiation area, and the recording unit acquires values of the contrast sensitivity when the observer looks at the visual targets presented by the visual target presenting unit and records the acquired values of the contrast sensitivity and positional information of the plurality of positions.

An illumination evaluation apparatus according to a third aspect of the present invention is the illumination evaluation apparatus according to the first or second aspect, further including: an illumination light measuring unit which measures at least one of illuminance, wavelength of the illumination light, color temperature of the illumination light, and flicker of the illumination light at the position where the visual target is presented by the visual target presenting unit. The recording unit records in relation to the acquired value of the contrast sensitivity, a value of the at least one of illuminance, wavelength of the illumination light, color temperature of the illumination light, and flicker of the illumination light acquired by the illumination light measuring unit.

An illumination evaluation apparatus according to a fourth aspect of the present invention is the illumination evaluation apparatus according to any one of the first to third aspect, further including: a time setting unit which sets as a number of acquisitions for values of at least the contrast sensitivity by the recording unit. The recording unit records an average of test results including the values of at least the contrast sensitivity acquired for the number of acquisitions set by the time setting unit.

An illumination evaluation apparatus according to a fifth aspect of the present invention is the illumination evaluation apparatus according to any one of the first to fourth aspects, further including: a reference value setting unit which sets as a reference value, a value of the contrast sensitivity of the observer acquired in a clear vision environment (a well-lit place). The recording unit calculates the difference between each value of the contrast sensitivity acquired by the observer and the reference value set by the reference value setting unit, and the visibility at the arbitrary position on the irradiation area is evaluated by a change in the contrast sensitivity from that in the clear vision environment, which corresponds to the calculated difference.

An illumination evaluation apparatus according to a sixth aspect of the present invention is the illumination evaluation apparatus according to any one of the first to fifth aspects, in which the value of the contrast sensitivity recorded by the recording unit and an illuminance value at the position where the value of the contrast sensitivity is acquired are recorded for each of the at least a light source.

An illumination evaluation apparatus according to a seventh aspect of the present invention is the illumination evaluation apparatus according to any one of the first to sixth embodiments, further including: a head position fixing unit which fixes the head position of the observer, in which the recording unit acquires and records a value of at least the contrast sensitivity with the distance between the irradiation area and the head position fixed.

An illumination evaluation method according to an eighth aspect of the present invention is an illumination evaluation method for evaluating an illumination environment by an arbitrary light source, the method comprising the steps of: projecting illumination light from the light source onto an irradiation area on which a visual target is visible at an arbitrary test position, the visual target being provided for acquiring contrast sensitivity of an observer looking at the test position; acquiring a value of the contrast sensitivity of the observer looking at the visual target presented at the test position in the state where the illumination light is projected from the light source onto the irradiation area; and using the acquired value of the contrast sensitivity to evaluate visibility at the arbitrary position on the irradiation area.

## BRIEF DESCRIPTION OF DRAWINGS

Fig. 1 is a block diagram illustrating the configuration of an illumination evaluation apparatus as an embodiment of the present invention.

Fig. 2 is a view illustrating a specific usage example of the illumination evaluation apparatus as the embodiment of the present invention.

Fig. 3 is a view illustrating another specific usage example of the illumination evaluation apparatus as the embodiment of the present invention.

Fig. 4 is a view illustrating an example of the form of visual targets presented to an observer in the illumination evaluation apparatus as the embodiment of the present invention.

Fig. 5 is a view illustrating another example of the form of visual targets presented to the observer in the illumination evaluation apparatus as the embodiment of the present invention.

Fig. 6 is a view illustrating still another example of the form of visual targets presented to the observer in the illumination evaluation apparatus as the embodiment of the present invention.

Fig. 7 is a view illustrating visual targets in the illumination evaluation apparatus as the embodiment of the present invention.

Fig. 8 is a view illustrating an example of a visual target presenting unit constituting an irradiation area in the illumination evaluation apparatus as the embodiment of the present invention.

Fig. 9 is a view illustrating an example of a visual target presenting unit constituting an irradiation area in the illumination evaluation apparatus as the embodiment of the present invention.

Fig. 10 is a diagram illustrating an example of test results by the illumination evaluation apparatus as the embodiment of the present invention, including plural contrast sensitivity values distributed.

Fig. 11 is a block diagram illustrating another configuration of the illumination evaluation apparatus as the embodiment of the present invention.

Fig. 12 is a view illustrating an illumination light measuring unit provided in the vicinity of a visual target in the illumination evaluation apparatus as the embodiment of the present invention.

Fig. 13 is a diagram illustrating an example of test results by the illumination evaluation apparatus as the embodiment of the present invention, including plural illuminance values distributed.

Fig. 14 is a diagram illustrating an example of test results by the illumination evaluation apparatus as the embodiment of the present invention, including plural wavelength spectrum values distributed.

Fig. 15 is a diagram illustrating an example of test results by the illumination evaluation apparatus as the embodiment of the present invention, including plural color temperature values distributed.

Fig. 16 is a block diagram illustrating still another configuration of the illumination evaluation apparatus as the embodiment of the present invention.

Fig. 17 is a diagram illustrating an example of test results by the illumination evaluation apparatus as the embodiment of the present invention, including plural average contrast sensitivities distributed.

Fig. 18 is a block diagram illustrating still another configuration of the illumination evaluation apparatus as the embodiment of the present invention.

Fig. 19 is a diagram illustrating an example of test results by the illumination evaluation apparatus as the embodiment of the present invention, including plural contrast sensitivity differences distributed.

Fig. 20 is a diagram illustrating an example of test results by the illumination evaluation apparatus as the embodiment of the present invention, showing distributions of contrast sensitivity values of different light sources.

Fig. 21 is a view showing a usage example of the illumination evaluation apparatus as the embodiment of the present invention in which the head is fixed.

Fig. 22 is a view showing another usage example of the illumination evaluation apparatus as the embodiment of the present invention in which the head is fixed.

## MODES FOR CARRYING OUT INVENTION

Hereinafter, a description is given of an embodiment of the present invention with reference to the drawings.

An illumination evaluation apparatus shown as an embodiment of the present invention is configured to evaluate an illumination environment (visibility) by an arbitrary light source. The illumination evaluation apparatus includes components illustrated in Fig. 1, for example. The illumination evaluation apparatus includes a visual target presenting unit 1 and a test result recording unit 2.

The target presenting unit 1 presents visual targets at arbitrary test positions on an irradiation area which is irradiated by illumination light emitted from a light source. The visual targets are used to measure the contrast sensitivity of an observer who sees the irradiation area. The visual target presenting unit 1 can be implemented by a plate, a sheet of paper, or the like on which the visual targets are drawn, for example.

The contrast sensitivity is one of visual performances to distinguish an obj ect which the observer is looking at from the background. The contrast sensitivity can be evaluated by measuring a visual target threshold which is the lightest visual target (with the lowest contrast) that can be recognized by the subject. The lower the contrast of the visual targets, the more difficult for the observer to see the visual target. Accordingly, when an observer can see low-contrast visual targets, it is determined that the observer can see well. Desirably, the contrast of a visual target presented to the observer is defined by the following formula 1 based on the maximum and minimum brightnesses of the visual target.

$$\text{Contrast} = (\text{Maximum Brightness} - \text{Minimum Brightness})/(\text{Maximum Brightness} + \text{Minimum Brightness}) \quad \text{(Formula 1)}$$

The form of visual targets presented to the observer, may be symbols such as the Landolt rings used in visual acuity tests or characters which have varied contrasts as illustrated in Fig. 2. Moreover, another form of visual targets presented to the observer may be striped patterns having varied contrasts as illustrated in Fig. 3. The display size of presented visual targets is desirably defined by a visual angle from the position of the subject's viewpoint. Moreover, the stripe width of the striped pattern is desirably defined by the number of cycles of a grating included in a visual angle of 1 degree (spatial frequency). Furthermore, the changes in the contrast of the striped patterns is desirably represented by gradation defined by sine wave gratings.

The test result recording unit 2 acquires contrast sensitivity values of the observer looking at the visual targets presented by the visual target presenting unit 1. The test result recording unit 2 records the obtained contrast sensitivity values and positional information of the test positions. The test result recording unit 2 includes a keyboard by which the contrast sensitivity values and test positions are inputted, a memory that records the contrast sensitivity values, and the like, for example. The test result recording unit 2 can be implemented by a personal computer, for example.

The illumination evaluation apparatus evaluates the visibility at an arbitrary position on the irradiation area by the contrast sensitivity values. For example, the illumination evaluation apparatus performs display, printing, or the like of the contrast sensitivity values recorded by the test result recording unit 2 in relation to the positions of the visual targets at which the contrast sensitivity values are obtained. The illumination evaluation apparatus can thereby evaluate the visibility at an arbitrary position on the irradiation area based on quantitative values, that is, the contrast sensitivity values.

Specifically, the visual target presenting unit 1 is used as illustrated in Figs. 5 and 6.

The visual target presenting unit 1 of Fig. 5 is provided for an irradiation area 12 on a table, for example. A light source 11 is installed over the irradiation area 12 of the visual target presenting unit 1. Illumination light L emitted from the light source 11 is projected onto the irradiation area 12. An observer P looks down at a visual target on the irradiation area 12 from a viewpoint S. In this state, the test result recording unit 2 measures and records the contrast sensitivity values of the observer P.

The visual target presenting unit 1 of Fig. 6 is provided for the irradiation area 12 on a wall surface, for example. The light source 11 is installed diagonally above the irradiation area 12 of the visual target presenting unit 1. The illumination light L emitted from the light source 11 is projected onto the irradiation area 12. The observer P faces the irradiation area 12 and looks at the visual targets on the irradiation area 12 from the viewpoint S. In this state, the test result recording unit 2 acquires and records the contrast sensitivity values of the observer P.

Visual targets 13 on the irradiation area 12 are provided as illustrated in Fig. 7, for example. The visual targets 13 include a reference visual target 13a. Each visual target 13 includes a striped pattern in any one of the upper and lower fields. The visual targets 13 are configured so that the contrast sensitivity values can be obtained when the observer selects one of the upper and lower fields of each visual target in which the observer can see the striped pattern. By the visual targets 13, each contrast sensitivity value is measured based on five-level test results. For example, No.1 striped pattern at the left end has the highest contrast, followed in order of contrast by No. 2, 3, 4, and 5 striped patterns. The test results are therefore obtained as: an observer with a high contrast sensitivity value can recognize the No. 5 striped pattern while an observer having a low contrast sensitivity value cannot recognize the No. 5 striped pattern. In the visual targets 13, five striped patterns having different contrasts are arranged in the horizontal direction. The visual targets 13 may have different sizes in accordance with viewpoint distance between the viewpoint S and the irradiation area 12.

Measurement points at which the visual targets 13 are placed are provided on the irradiation area 12 as illustrated in Fig. 8, for example. The measurement points are set to arbitrary positions on the irradiation area 12. On the irradiation area 12 of Fig. 8, a measurement point A is set to the center of the irradiation area 12, and measurement points B to Q are provided around the center in a spiral fashion. The irradiation evaluation apparatus may employ either plural measurement points or a single measurement point. In the case of using plural measurement points, the irradiation evaluation apparatus can provide pairs of measurement points and contrast sensitivity values as the test results. In the case of using a single measurement point, the irradiation evaluation apparatus can provide a contrast sensitivity value at only one arbitrary point as the test results.

The target presenting unit 1 is configured so that the visual targets 13A to 13Q are respectively provided at the measurement points A to Q on the irradiation area 12 as illustrated in Fig. 9.

The contrast sensitivity values and test positions, using the visual target presenting unit 1, are recorded by the test result recording unit 2. The test result recording unit 2 can then display a test result 20 representing contrast sensitivity values A to Q distributed at the respective test positions as illustrated in Fig. 10. The contrast sensitivity values A to Q are measurement results of contrast sensitivity values obtained when an observer looks at the visual targets 13A to 13Q illustrated in Fig. 9. With reference to the test result 20 of Fig. 10, the contrast sensitivity value A (test value A) at the central measurement point A is 5 by the brightness of the illumination light projected from the light source 11 onto the irradiation area 12 and the like. The contrast sensitivity value J (test value J) at the measurement point J distant from the center is 3, for example.

In the configuration described above, to examine the contrast sensitivity value at each arbitrary position on the irradiation area 12, the light source 11 is turned on at first. In this process, illumination light is projected from the light source 11 onto the visual target presenting unit 1 including the irradiation area 12 on which the visual targets 13 are made visible at arbitrary test positions for measuring the contrast sensitivity of the observer looking at the test positions. Next, in the state where the illumination light is being projected from the light source 11 onto the irradiation area 12, the test result recording unit 2 measures the contrast sensitivity values A to Q at the time when the observer looks at the visual targets at the test positions A to Q, respectively. The test result recording unit 2 records the measured contrast sensitivity values A to Q and the positions A to Q of the visual targets. The illumination evaluation apparatus can therefore display map data in which the visibility at arbitrary positions on the irradiation area 12 by the light source 11 is evaluated by the contrast sensitivity values as illustrated in Fig. 10.

As described above, according to the illumination evaluation apparatus, in an illumination environment on the irradiation area 12 by the arbitrary light source 11, the contrast sensitivity values at arbitrary places are measured, and the measured values are mapped. The illumination evaluation apparatus can thus represent the visibility of the illumination environment quantitatively and objectively.

According to the illumination evaluation apparatus, the contrast sensitivity values are displayed at the positions corresponding the respective measurement points. The visibility on the irradiation area 12 by illumination light emitted from the light source 11 can be therefore evaluated as the distribution of contrast sensitivity values.

In addition to the aforementioned configuration, the above-described illumination evaluation apparatus may include an illumination light measuring unit 3 as illustrated in Fig. 11. The illumination light measuring unit 3 measures at least one of illuminance, wavelength of the illumination light, color temperature of the illumination light, and flicker of the illumination light at the test positions where the visual targets 13 are presented by the visual target presenting unit 1. The illumination light measuring unit 3 is composed of an optical sensor installed near the visual target 13 provided at each measurement point as illustrated in Fig. 12, for example.

The test result recording unit 2 records at least one of illuminance, wavelength of the illumination light, color temperature of the illumination light, and flicker of the illumination light which is measured by the illumination light measuring unit 3 in relation to the corresponding contrast sensitivity values and positional information.

When the illuminance is measured by the illumination light measuring unit 3, the thus-configured illumination evaluation apparatus can obtain a test result 21 as illustrated in Fig. 13, for example. The test result 21 shows illuminances A to Q which are measured by the illumination light measuring unit 3 and are distributed at the measurement points A to Q. The illuminances A to Q are displayed at the positions corresponding to the measurement points A to Q, respectively. The illumination evaluation apparatus can therefore display the distribution of the illuminances A to Q in addition to the distribution of the contrast sensitivity values A to Q at the measurement points A to Q on the irradiation area 12. With reference to the test result 21 of Fig. 13, the illuminance at the central measurement point A is 100 lx by the brightness of the illumination light projected from the light source 11 onto the irradiation area 12. The illuminance J at the measurement point J distant from the center is 30 lx, for example.

The illumination evaluation apparatus can record the relation between the illuminances by the light source 11 and the contrast sensitivity values based on the test result 20 showing the distribution of the contrast sensitivity values A to Q and the test result 21 showing the distribution of the illuminances A to Q. The visibility of the light source 11 can be therefore evaluated more quantitatively and objectively.

When the wavelength is measured by the illumination light measuring unit 3, the illumination evaluation apparatus can obtain a test result 22 as illustrated in Fig. 14, for example. The test result 22 shows wavelength spectrum values A to Q which are measured by the illumination light measuring unit 3 and are distributed at the measurement points A to Q. The wavelength spectrum values A to Q are displayed at the positions corresponding to the measurement points A to Q, respectively. The illumination evaluation apparatus can therefore display the distribution of the wavelength spectrum values A to Q in addition to the distribution of the contrast sensitivity values A to Q at the measurement points A to Q on the irradiation area 12. With reference to the test result 22 of Fig. 14, the wavelength spectrum value A at the central measurement point A is 600 nm by the illumination light projected from the light source 11 onto the irradiation area 12. The wavelength spectrum value J at the measurement point J distant from the center is 580 nm, for example.

The illumination evaluation apparatus can record the relation between the wavelength spectrum values by the light source 11 and the contrast sensitivity values based on the test result 20 showing the distribution of the contrast sensitivity values A to Q and the test result 22 showing the distribution of the wavelength spectrum values A to Q. The visibility of the light source 11 can be therefore evaluated more quantitatively and objectively.

When the color temperature is measured by the illumination light measuring unit 3, the illumination evaluation apparatus can obtain a test result 23 as illustrated in Fig. 15, for example. The test result 23 shows color temperatures A to Q corresponding to the measurement points A to Q which are measured by the illumination light measuring unit 3. The color temperatures A to Q are displayed at the positions corresponding to the measurement points A to Q, respectively. The illumination evaluation apparatus can therefore display the distribution of the color temperatures A to Q in addition to the distribution of the contrast sensitivity values A to Q at the measurement points A to Q on the irradiation area 12. With reference to the test result 23 of Fig. 15, the color temperature A at the central measurement point A is 5000 K by the illumination light projected from the light source 11 onto the irradiation area 12. The color temperature J at the measurement point J distant from the center is 4800 K, for example.

The illumination evaluation apparatus can record the relation between the color temperatures by the light source 11 and the contrast sensitivity values based on the test result 20 showing the distribution of the contrast sensitivity values A to Q and the test result 23 showing the distribution of the wavelength spectrum values A to Q. The visibility of the light source 11 can be therefore evaluated more quantitatively and objectively.

In addition to the aforementioned configuration, the aforementioned illumination evaluation apparatus may include a test number of times setting unit 4 as illustrated in Fig. 16. The test number of times setting unit 4 sets at least the number of times of tests (the number of times of acquisitions) for contrast sensitivity values to be performed by the test result recording unit 2. The test number of times setting unit 4 accepts the number of times of tests inputted in accordance with the user's operation, for example, and sets the number of times of tests for contrast sensitivity values by the test result recording unit 2. The test result recording unit 2 measures at least the contrast sensitivity values for the number of times of acquisitions set by the illumination light measuring unit 3. The test result recording unit 2 averages the plural contrast sensitivity values measured. The test result recording unit 2 records the averaged contrast sensitivity value as the test result.

The thus-configured illumination evaluation apparatus can provide a test result 24 including contrast sensitivity value averages A to Q which are obtained by averaging the contrast sensitivity values measured in the set number of times of tests at the respective measurement points A to Q as illustrated in Fig. 17. The contrast sensitivity value averages A to Q are displayed at the positions corresponding to the measurement points A to Q, respectively. With reference to the test result 24 of Fig. 17, the contrast sensitivity value average A at the central measurement point A is 5 by the brightness or the like of the illumination light projected from the light source 11 onto the irradiation area 12. The contrast sensitivity value average J at the measurement point J distant from the center is 3, for example.

Accordingly, the illumination evaluation apparatus can display the test result 24 showing the distribution of the contrast sensitivity value averages A to Q to evaluate the visibility of the light source 11 quantitatively and objectively. Moreover, the illumination evaluation apparatus averages the test results, thus increasing the reproducibility of the test results and reducing the variations of the test results.

In addition to the aforementioned configuration, the above-described illumination evaluation apparatus may include a reference value setting unit 6 and a difference recording unit 5 as illustrated in Fig. 18. The reference value setting unit 6 sets as a reference value, the contrast sensitivity value of an observer measured in a clear vision environment. The clear vision environment refers to an illumination environment where objects in the field of sight can be clearly seen. The reference value setting unit 6 sets the reference value to a contrast sensitivity value measured in the clear vision environment where it is bright around the observer P. The reference value setting unit 6 may be a contrast sensitivity meter which measures the contrast sensitivity value in the clear vision environment, for example. The reference value setting unit 6 may be an operation device through which the contrast sensitivity value in the clear vision environment is inputted.

The difference recording unit 5 calculates and records the difference between each contrast sensitivity value measured by the observer and the reference value set by the reference value setting unit 6. The difference recording unit 5 may be composed of the processing IC and memory same as those of the test result recording unit 2.

The thus-configured illumination evaluation apparatus can provide a test result 25 including contrast sensitivity differences A to Q recorded by the difference recording unit 5 for the respective measurement points A to Q as illustrated in Fig. 19. The test result 25 shows the contrast sensitivity differences A to Q corresponding to the measurement points A to Q, respectively. The contrast sensitivity differences A to Q are displayed at the positions corresponding to the measurement points A to Q, respectively. With reference to the test result 25 of Fig. 19, the brightness of the illumination light projected from the light source 11 onto the irradiation area 12 is equal to that in the clear vision environment, and the contrast sensitivity difference A at the central measurement point A is 0. At the measurement point J distant from the center, for example, the contrast sensitivity difference J is -2 for the reasons including the brightness lower than the clear vision environment.

The illumination evaluation apparatus can therefore evaluate the visibility at an arbitrary test position on the irradiation area 12 by the light source 11 based on the variations in contrast sensitivity values from the clear vision environment (corresponding to the calculated differences). In other words, the illumination evaluation apparatus can measure reduction in the contrast sensitivity value from the clear vision environment to evaluate the environment on the irradiation area 12 relative to the clear vision environment.

The aforementioned illumination evaluation apparatus may evaluate illumination light projected onto the irradiation area 12 from different light sources 11 and store the test result of each light source 11. The illumination evaluation apparatus records for each light source 11, the contrast sensitivity values recorded by the test result recording unit 2 and the positions at which the contrast sensitivity values are tested.

When the different light sources include light sources (1), (2), and (3), for example, the test result recording unit 2 measures contrast sensitivity values A to Q at the measurement points A to Q for the light sources (1), (2), and (3). The test result recording unit 2 stores the positions of the measurement points A to Q and the contrast sensitivity values A to Q for each light source (1), (2), and (3).

The thus-configured illumination evaluation apparatus can provide a test result 26 including the contrast sensitivity values A to Q of the light sources (1), (2), and (3) recorded for the measurement points A to Q. The test result 26 shows the contrast sensitivity values A to Q corresponding to the measurement points A to Q for each light source (1), (2), and (3). The contrast sensitivity values A to Q are respectively displayed at the positions corresponding to the measurement points A to Q for each light source (1), (2), and (3). The test result recording unit 2 can record the measured values of any of the illuminance, spectrum, color temperature, and frequency for each light source (not shown).

Herein, the different light sources 11 may be of different types or may be of a same type but have different specifications. The different light sources 11 may include metal halide lamps and ultrahigh pressure mercury lamps used in projectors, xenon lamps, and halogen lamps for illumination, for example. Metal halide lamps have a color temperature of about 6500 to 7500 K. Ultrahigh pressure mercury lamps have a color temperature of about 9000 K. Xenon lamps have a color temperature of about 6000 K. Halogen lamps for illumination have a color temperature of about 3400 K. Moreover, the different types of light sources 11 may include a type of incandescent halogen lamps using thermal radiation and a type of ultrahigh pressure mercury lamps using discharge optical emission, which are different types from each other. Furthermore, the light sources which are of a same type but have different specifications are LEDs having different wavelength spectra or the like.

According to the illumination evaluation apparatus, in illumination evaluation using plural light sources 11, the evaluation results of the individual light sources 11 can be recorded and compared with each other. The illumination evaluation apparatus can therefore objectively evaluate visibility at an arbitrary position on the irradiation area 12 by the light sources 11 for each light source 11.

Furthermore, as illustrated in Figs. 21 and 22, preferably, the aforementioned illumination evaluation apparatus includes a chin rest 30 as a head position fixing unit by which the observer's head position is fixed. In the illumination evaluation apparatus, the test result recording unit 2 measures and records at least the contrast sensitivity values after the distance between the irradiation area 12 and the head position is fixed by the chin rest 30.

Herein, the size of the testing visual targets displayed at the contrast sensitivity test is determined by the viewpoint distance. Accordingly, to increase the reliability of contrast sensitivity values, it is important to maintain the viewpoint distance between the viewpoint S of the observer P and the irradiation area 12. The illumination evaluation apparatus includes the tin rest 30, and the head of the observer P is fixed by the tin rest 30 for fixing the viewpoint S. The viewpoint distance between the viewpoint S and the irradiation area 12 can be maintained.

The aforementioned embodiment is just an example of the present invention. The present invention is therefore not limited by the aforementioned embodiment and, in addition to the embodiment, can be variously changed in accordance with the design and the like without departing from the technical idea according to the present invention.

The entire contents of Japanese Patent Application Publication No. 2012-128324 (filed on: 5 June 2012) are incorporated herein by reference.

## INDUSTRIAL APPLICABILITY

According to the present invention, the visibility at an arbitrary position on the irradiation area is evaluated by contrast sensitivity values. Accordingly, the illumination environment can be represented quantitatively.

## EXPLANATION OF REFERENCIAL SYMBOLS

| | |
|---|---|
| 1 | VISUAL TARGET PRESENTING UNIT |
| 2 | TEST RESULT RECORDING UNIT |
| 3 | ILLUMINATION LIGHT MEASURING UNIT |
| 4 | TEST NUMBER OF TIMES SETTING UNIT |
| 5 | DIFFERENCE RECORDING UNIT |
| 6 | REFERENTIAL VALUE SETTING UNIT |
| 11 | LIGHT SOURCE |
| 12 | IRRADIATION AREA |
| 13 | VISUAL TARGET |

## Claims

**1.** An illumination evaluation apparatus evaluating an illumination environment by at least an arbitrary light source, the apparatus comprising:

a visual target presenting unit which presents a visual target at an arbitrary test position on an irradiation area irradiated by illumination light emitted from the at least a light source, the visual target being provided for acquiring contrast sensitivity of an observer looking at the irradiation area; and
a recording unit which acquires a value of the contrast sensitivity when the observer looks at the visual target presented by the visual target presenting unit, wherein
the acquired value of the contrast sensitivity is used to evaluate visibility at the arbitrary position on the irradiation area.

2. The illumination evaluation apparatus according to claim 1, wherein
the visual target presenting unit presents the visual target for acquiring the contrast sensitivity at each of a plurality of arbitrary positions on the irradiation area, and
the recording unit acquires values of the contrast sensitivity when the observer looks at the visual targets presented by the visual target presenting unit and records the acquired values of the contrast sensitivity and positional information of the plurality of positions.

3. The illumination evaluation apparatus according to claim 1 or 2, further comprising:

an illumination light measuring unit which measures at least one of illuminance, wavelength of the illumination light, color temperature of the illumination light, and flicker of the illumination light at the position where the visual target is presented by the visual target presenting unit, wherein
the recording unit records in relation to the acquired value of the contrast sensitivity, a value of the at least one of illuminance, wavelength of the illumination light, color temperature of the illumination light, and flicker of the illumination light acquired by the illumination light measuring unit.

4. The illumination evaluation apparatus according to any one of claims 1 to 3, further comprising:

a time setting unit which sets as a number of acquisitions for values of at least the contrast sensitivity by the recording unit, wherein
the recording unit records an average of test results including the values of at least the contrast sensitivity acquired for the number of acquisitions set by the time setting unit.

5. The illumination evaluation apparatus according to any one of claims 1 to 4, further comprising:

a reference value setting unit which sets as a reference value, a value of the contrast sensitivity of the observer acquired in a clear vision environment, wherein
the recording unit calculates the difference between each value of the contrast sensitivity acquired by the observer and the reference value set by the reference value setting unit, and
the visibility at the arbitrary position on the irradiation area is evaluated by a change in the contrast sensitivity from that in the clear vision environment, which corresponds to the calculated difference.

6. The illumination evaluation apparatus according to any one of claims 1 to 5, wherein the value of the contrast sensitivity recorded by the recording unit and an illuminance value at the position where the value of the contrast sensitivity is acquired are recorded for each of the at least a light source.

7. The illumination evaluation apparatus according to any one of claims 1 to 6, further comprising:

a head position fixing unit which fixes the head position of the observer, wherein
the recording unit acquires and records a value of at least the contrast sensitivity with the distance of the irradiation area and the head position fixed.

8. An illumination evaluation method for evaluating an illumination environment by an arbitrary light source, the method comprising the steps of:

projecting illumination light from the light source onto an irradiation area on which a visual target is visible at an arbitrary test position, the visual target being provided for acquiring contrast sensitivity of an observer looking at the test position;
acquiring a value of the contrast sensitivity of the observer looking at the visual target presented at the test position in the state where the illumination light is projected from the light source onto the irradiation area; and
using the acquired value of the contrast sensitivity to evaluate visibility at the arbitrary position on the irradiation area.

FIG. 1

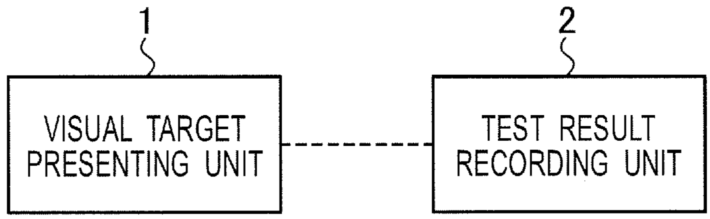
1
VISUAL TARGET PRESENTING UNIT
2
TEST RESULT RECORDING UNIT

FIG. 2

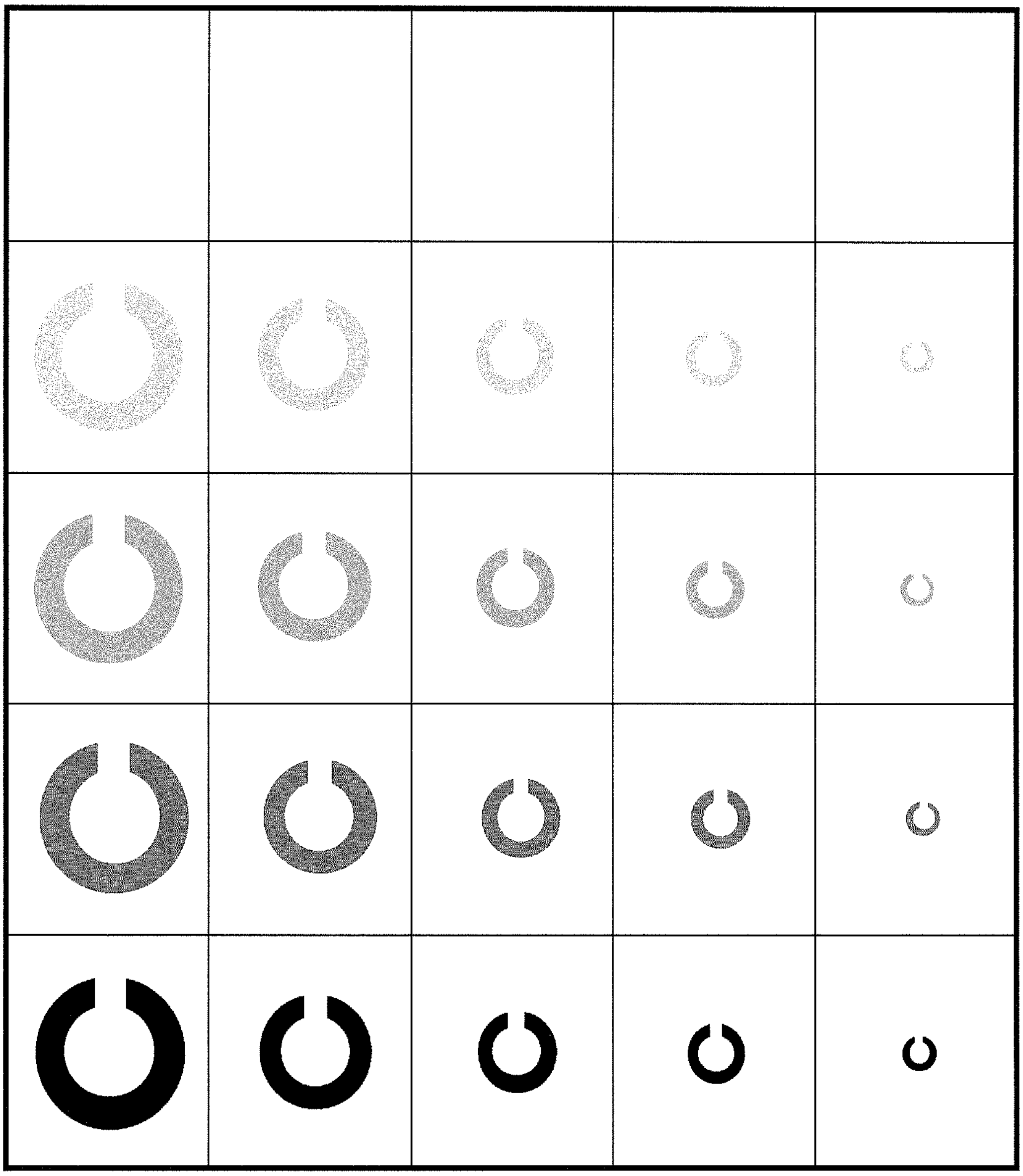

FIG. 3

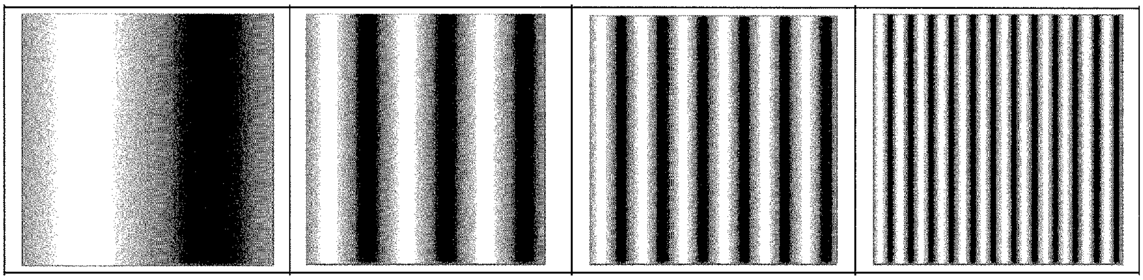

FIG. 4

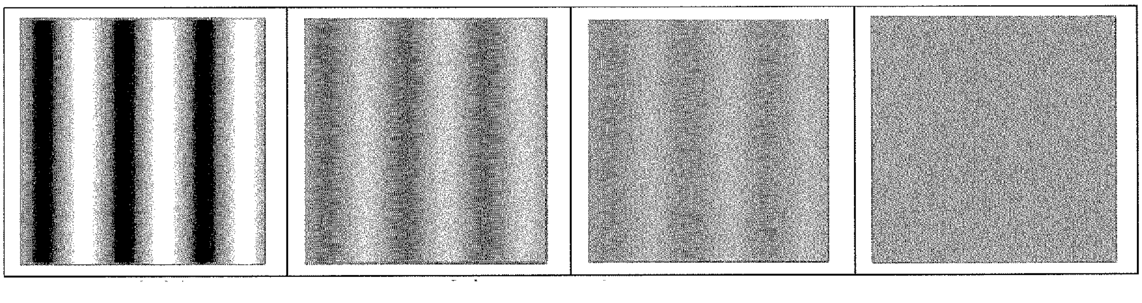

FIG. 5

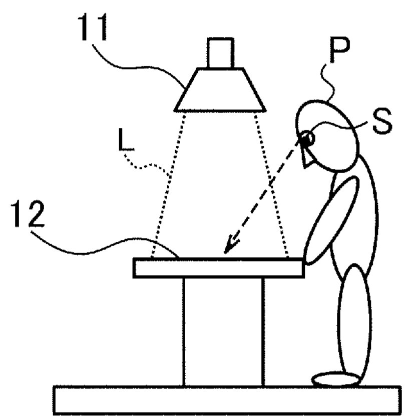


FIG. 6

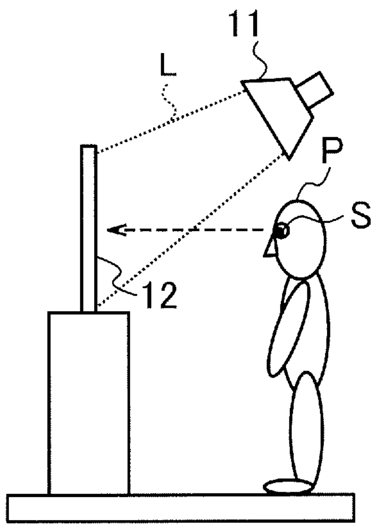

FIG. 7

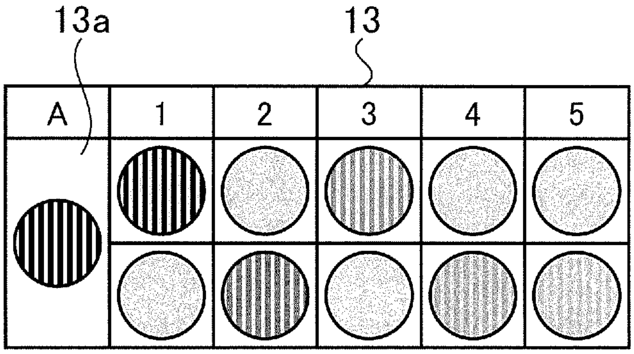


FIG. 8

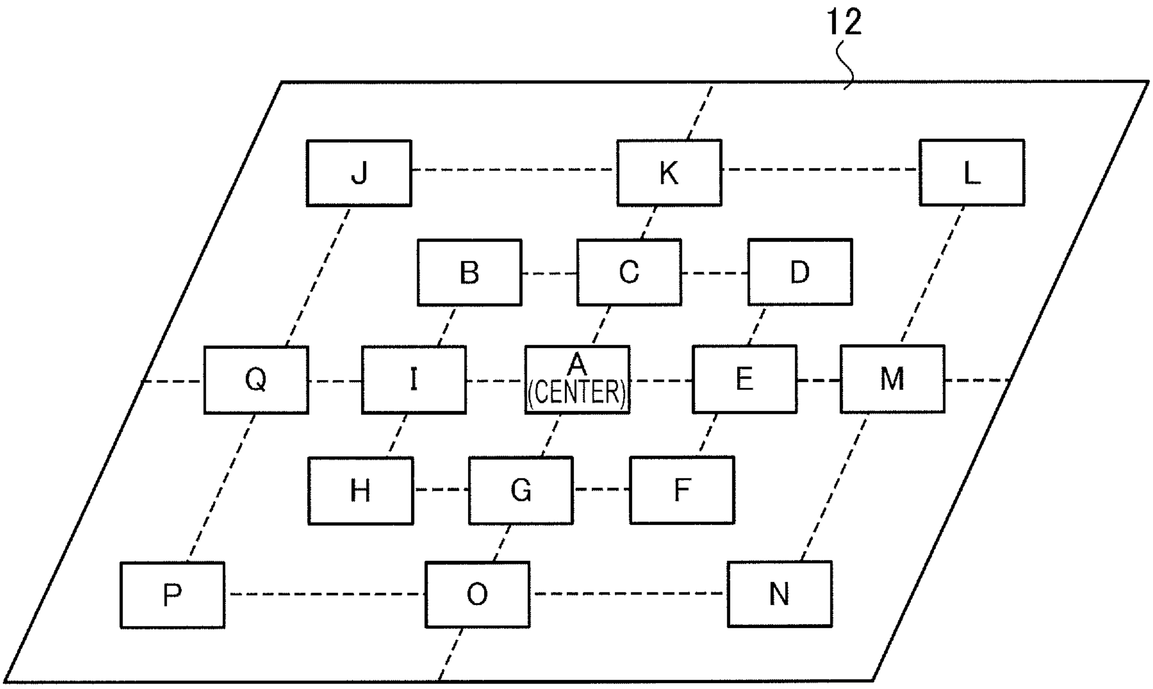

FIG. 9

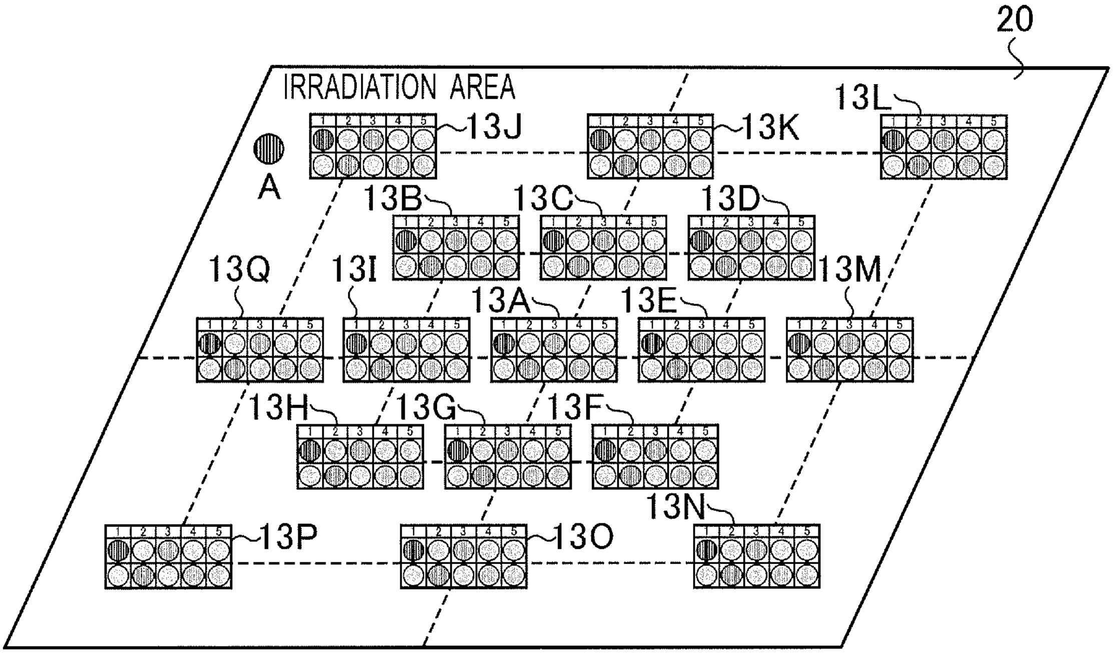
20
IRRADIATION AREA
A
13J
13K
13L
13B
13C
13D
13Q
13I
13A
13E
13M
13H
13G
13F
13N
13P
13O

FIG. 10

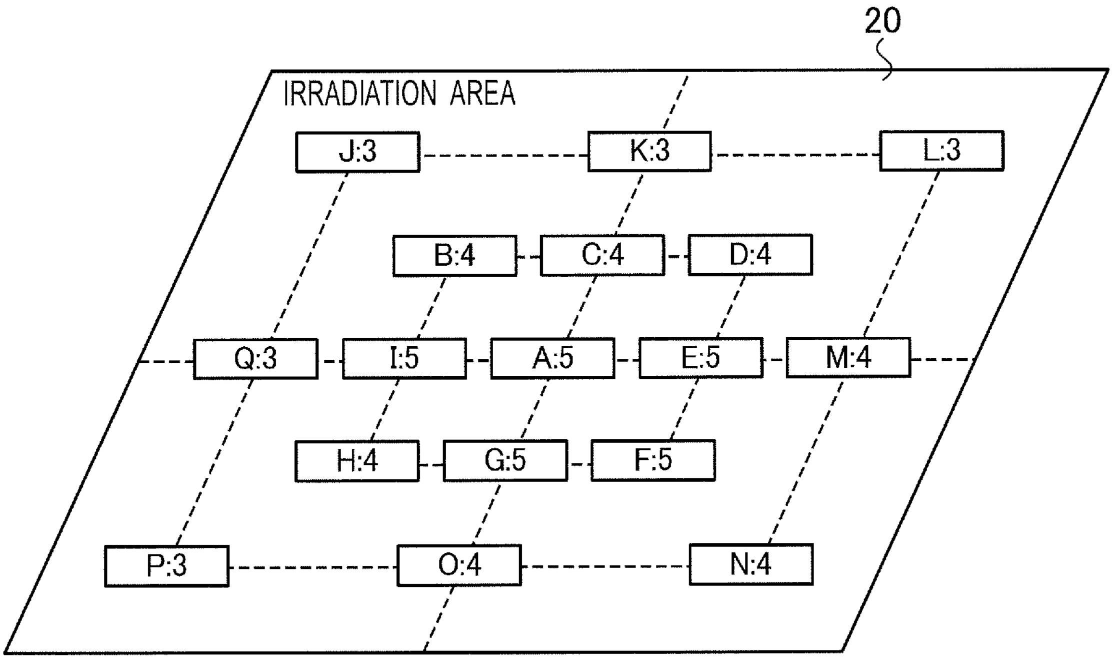
20
IRRADIATION AREA
J:3
K:3
L:3
B:4
C:4
D:4
Q:3
I:5
A:5
E:5
M:4
H:4
G:5
F:5
P:3
O:4
N:4

FIG. 11

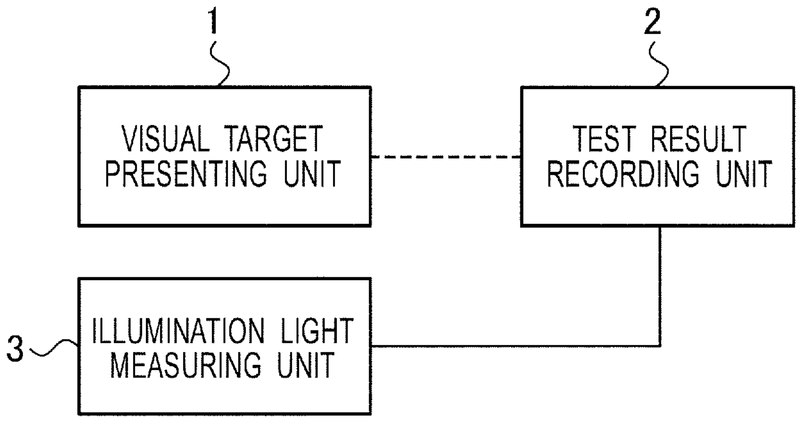
1
2
VISUAL TARGET PRESENTING UNIT
TEST RESULT RECORDING UNIT
3
ILLUMINATION LIGHT MEASURING UNIT

FIG. 12

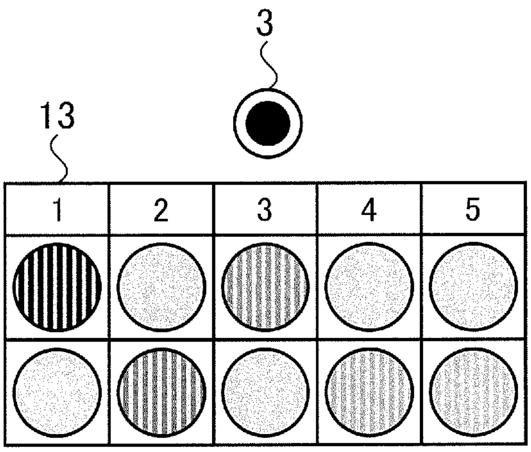
3
13
1
2
3
4
5

FIG. 13

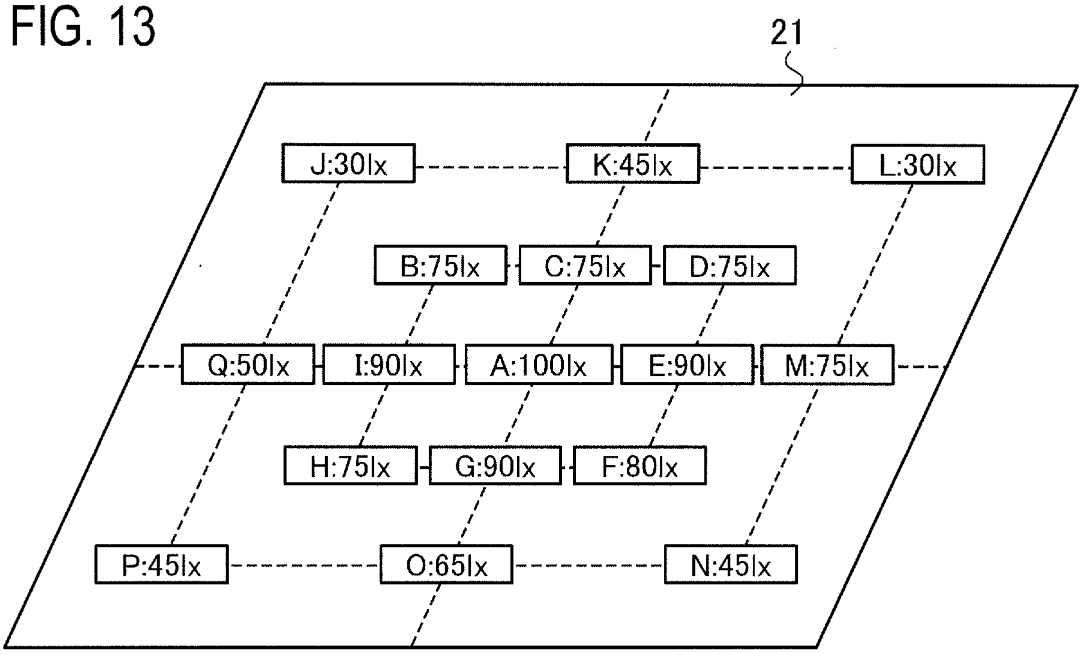
21
J:30lx
K:45lx
L:30lx
B:75lx
C:75lx
D:75lx
Q:50lx
I:90lx
A:100lx
E:90lx
M:75lx
H:75lx
G:90lx
F:80lx
P:45lx
O:65lx
N:45lx

FIG. 14

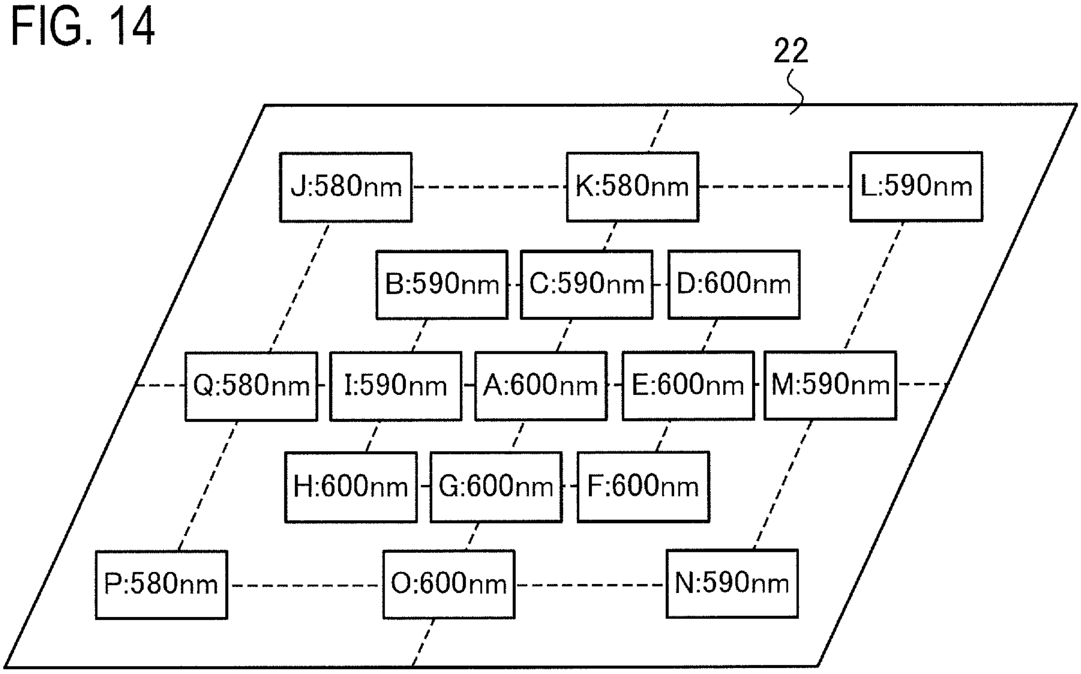
22
J:580nm
K:580nm
L:590nm
B:590nm
C:590nm
D:600nm
Q:580nm
I:590nm
A:600nm
E:600nm
M:590nm
H:600nm
G:600nm
F:600nm
P:580nm
O:600nm
N:590nm

FIG. 15

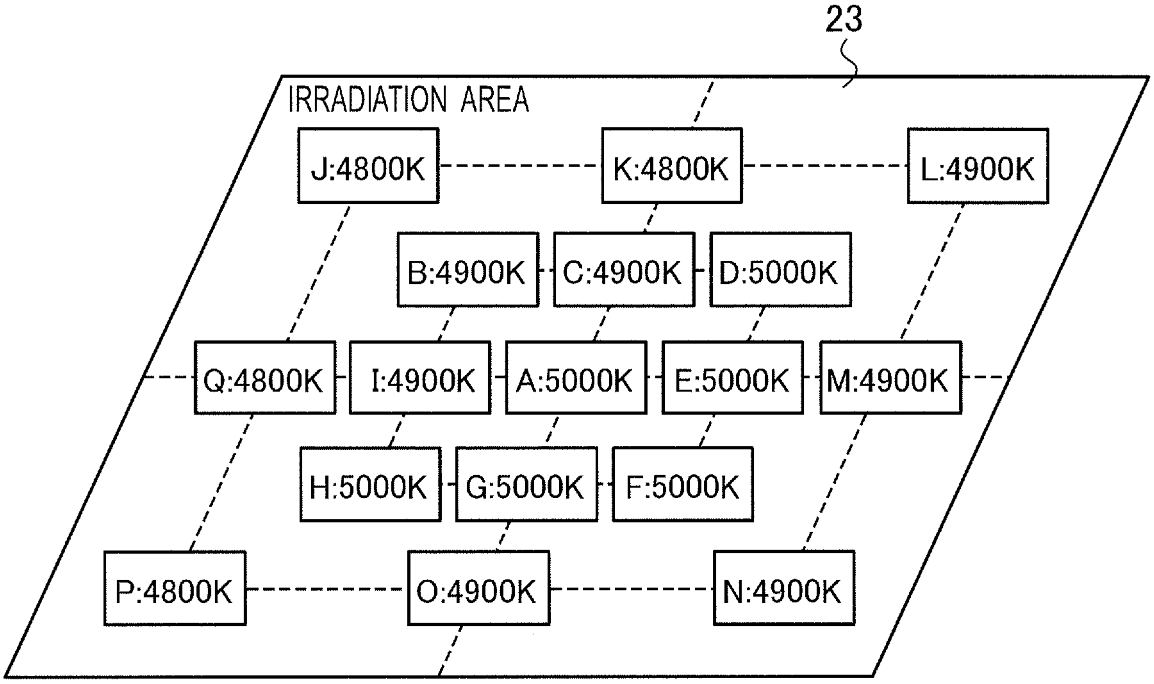
23
IRRADIATION AREA
J:4800K
K:4800K
L:4900K
B:4900K
C:4900K
D:5000K
Q:4800K
I:4900K
A:5000K
E:5000K
M:4900K
H:5000K
G:5000K
F:5000K
P:4800K
O:4900K
N:4900K

FIG. 16

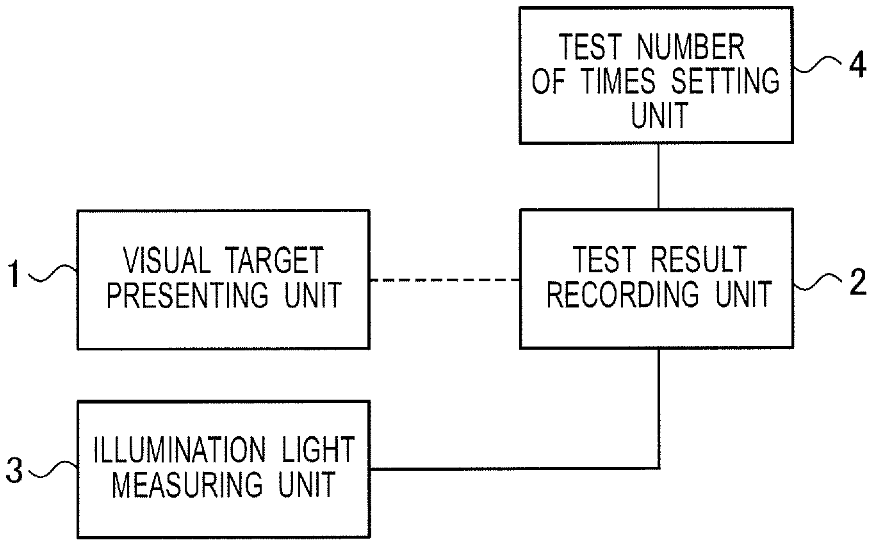
TEST NUMBER OF TIMES SETTING UNIT
4
VISUAL TARGET PRESENTING UNIT
1
TEST RESULT RECORDING UNIT
2
ILLUMINATION LIGHT MEASURING UNIT
3

FIG. 17

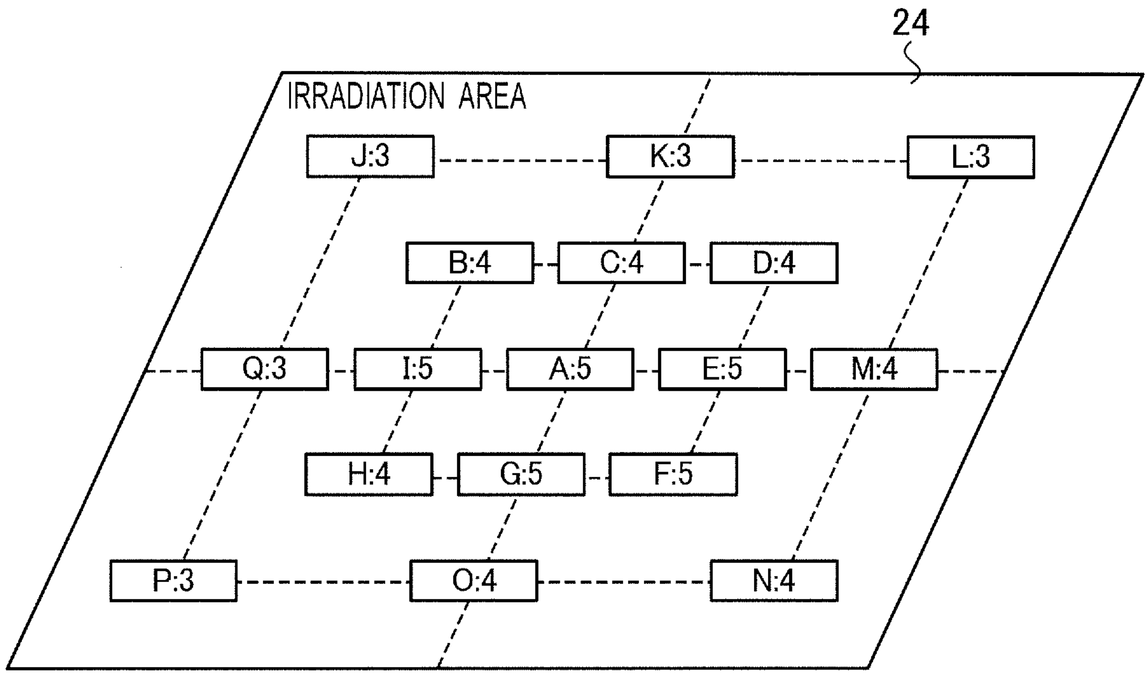
24
IRRADIATION AREA
J:3
K:3
L:3
B:4
C:4
D:4
Q:3
I:5
A:5
E:5
M:4
H:4
G:5
F:5
P:3
O:4
N:4

FIG. 18

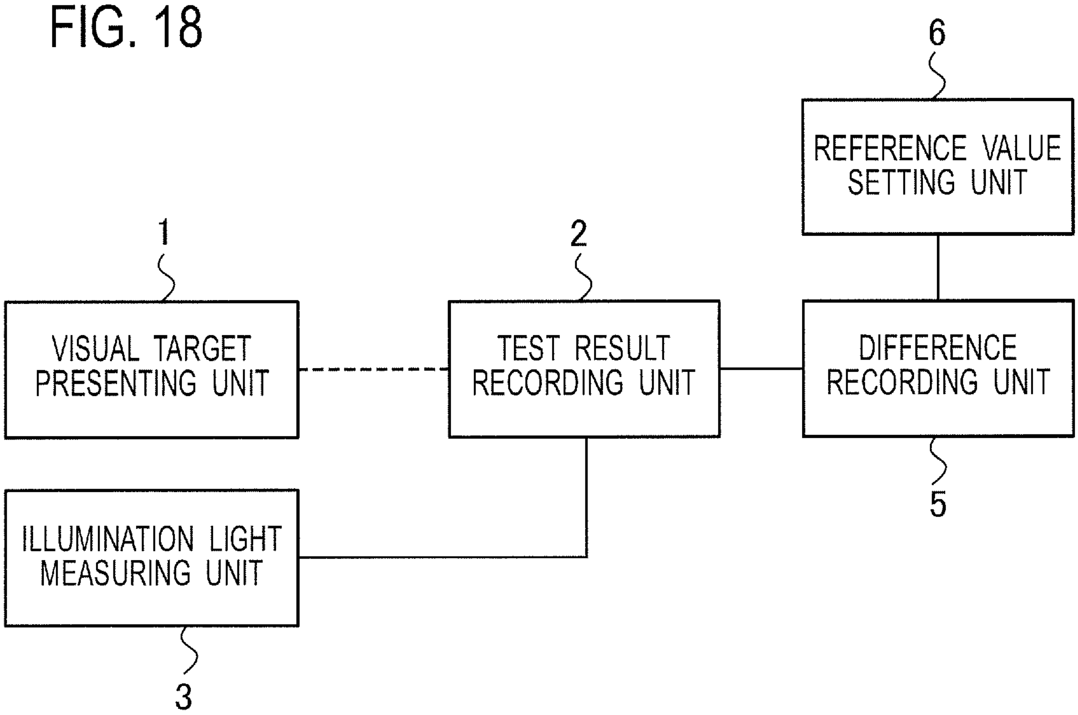
6
REFERENCE VALUE SETTING UNIT
1
VISUAL TARGET PRESENTING UNIT
2
TEST RESULT RECORDING UNIT
DIFFERENCE RECORDING UNIT
5
ILLUMINATION LIGHT MEASURING UNIT
3

FIG. 19

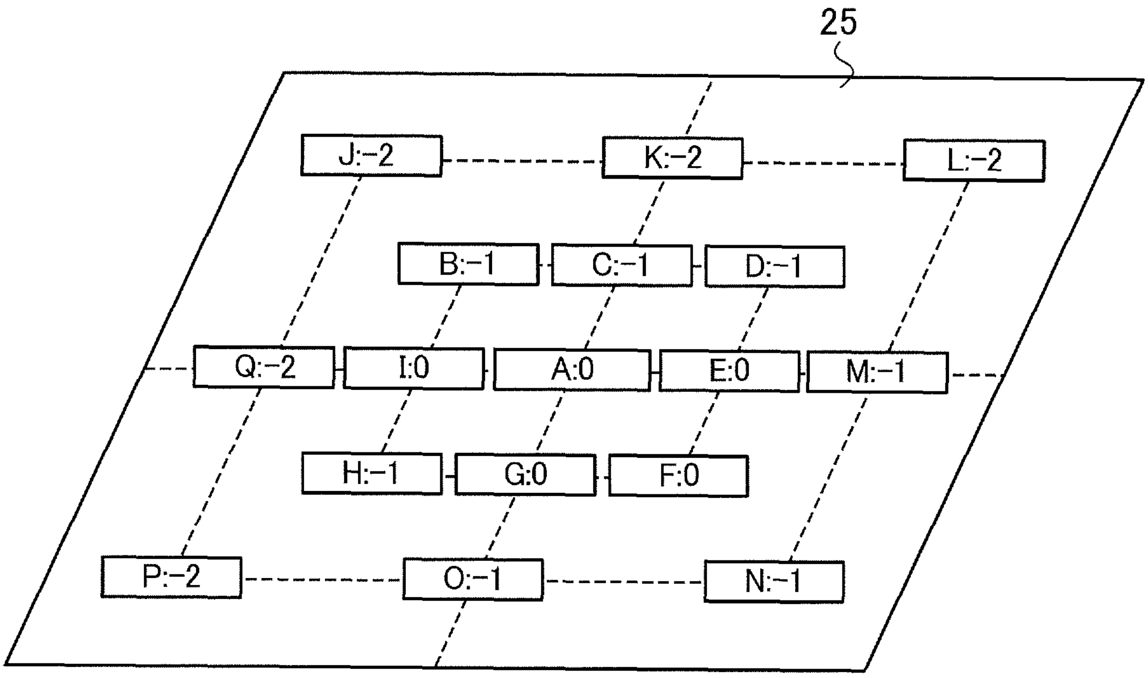

FIG. 20

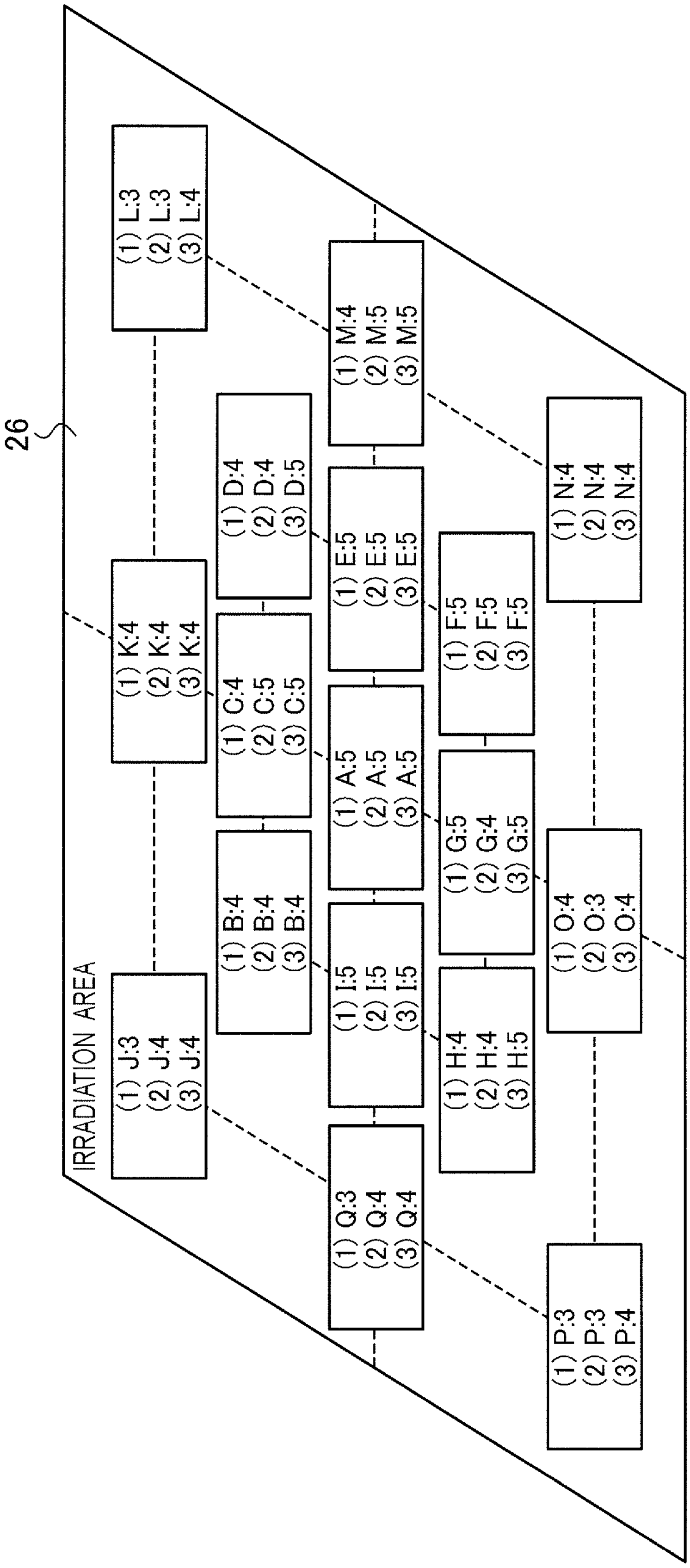
26
IRRADIATION AREA
(1) J:3 (2) J:4 (3) J:4
(1) K:4 (2) K:4 (3) K:4
(1) L:3 (2) L:3 (3) L:4
(1) B:4 (2) B:4 (3) B:4
(1) C:4 (2) C:5 (3) C:5
(1) D:4 (2) D:4 (3) D:5
(1) Q:3 (2) Q:4 (3) Q:4
(1) I:5 (2) I:5 (3) I:5
(1) A:5 (2) A:5 (3) A:5
(1) E:5 (2) E:5 (3) E:5
(1) M:4 (2) M:5 (3) M:5
(1) H:4 (2) H:4 (3) H:5
(1) G:5 (2) G:4 (3) G:5
(1) F:5 (2) F:5 (3) F:5
(1) P:3 (2) P:3 (3) P:4
(1) O:4 (2) O:3 (3) O:4
(1) N:4 (2) N:4 (3) N:4

FIG. 21

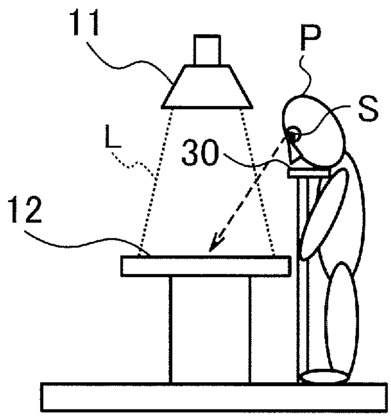


FIG. 22

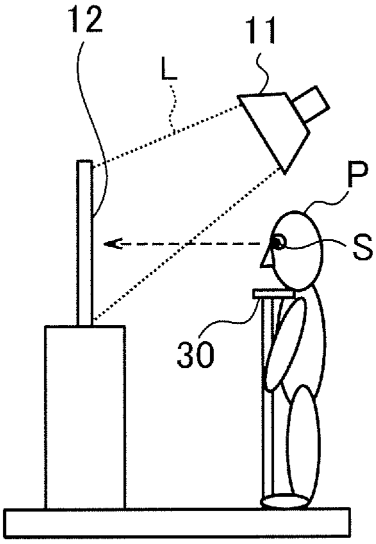

**INTERNATIONAL SEARCH REPORT**

International application No.
PCT/JP2013/000734

A. CLASSIFICATION OF SUBJECT MATTER
*GG01M11/00*(2006.01)i, *G01J1/02*(2006.01)i, *G01J3/50*(2006.01)i, *G01J5/60*(2006.01)i, *A61B3/028*(2006.01)i, *H05B37/02*(2006.01)i

According to International Patent Classification (IPC) or to both national classification and IPC

B. FIELDS SEARCHED

Minimum documentation searched (classification system followed by classification symbols)
G01M11/00, G01J1/00-1/02, G01J1/42, G01J3/46-3/52, G01J5/60, A61B3/028-3/032, H05B37/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | Yutaka TAKEZAKI, "Study of illumination by a desk lamp", Bulletin of The Faculty of School Education, Hiroshima University, 25 December 1988 (25.12.1988), Dai II Bu, vol.11, pages 155 to 161 | 1-8 |
| Y | Shodiq W Alwi et al., "Research on Comfortable Lighting Environment For Presentation", Architectural Institute of Japan 2003 Nendo Taikai (Tokai) Gakujutsu Koen Kogaishu Kankyo Kogaku I, 30 July 2003 (30.07.2003), separate vol. D-1, pages 327 to 328 | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 May, 2013 (06.05.13) | 28 May, 2013 (28.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/000734

C (Continuation). DOCUMENTS CONSIDERED TO BE RELEVANT

| Category* | Citation of document, with indication, where appropriate, of the relevant passages | Relevant to claim No. |
|---|---|---|
| Y | JP 09-147082 A (Sumitomo Heavy Industries, Ltd.), 06 June 1997 (06.06.1997), paragraphs [0015], [0026] to [0027]; fig. 3 to 4 (Family: none) | 5,7 |
| Y | WO 2010/064492 A1 (Panasonic Electric Works Co., Ltd.), 10 June 2010 (10.06.2010), paragraph [0039]; fig. 1 & JP 2009-273869 A & US 2011/0205491 A1 & EP 2353495 A1 | 7 |
| A | JP 2012-064541 A (Toshiba Lighting & Technology Corp.), 29 March 2012 (29.03.2012), entire text; fig. 1 to 5 (Family: none) | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3623401 B
- JP 3850186 B
- JP 2012128324 A